(19) Europäisches Patentamt
European Patent Office
Office européen des brevets

(11) **EP 1 893 109 B1**

(12) **EUROPEAN PATENT SPECIFICATION**

(45) Date of publication and mention
of the grant of the patent:
**04.10.2017 Bulletin 2017/40**

(21) Application number: **06773170.3**

(22) Date of filing: **14.06.2006**

(51) Int Cl.:
***A61B 17/58*** (2006.01)

(86) International application number:
**PCT/US2006/023196**

(87) International publication number:
**WO 2006/138398 (28.12.2006 Gazette 2006/52)**

(54) **TOOL AND TECHNIQUE FOR CONTINUALLY DELIVERING AN ORTHOPAEDIC PASTE**

WERKZEUG UND TECHNIK ZUR KONTINUIERLICHEN ABGABE EINER ORTHOPÄDISCHEN PASTE

OUTIL DE DISTRIBUTION DE PATE ORTHOPEDIQUE, PROCEDE PERMETTANT DE DISTRIBUER LA PATE EN CONTINU ET DISPOSITIFS ET METHODES FACILITANT LA DISTRIBUTION DE LA PATE ORTHOPEDIQUE

(84) Designated Contracting States:
**AT BE BG CH CY CZ DE DK EE ES FI FR GB GR HU IE IS IT LI LT LU LV MC NL PL PT RO SE SI SK TR**

(30) Priority: **15.06.2005 US 152170**
**02.09.2005 US 713851 P**
**30.01.2006 US 763134 P**

(43) Date of publication of application:
**05.03.2008 Bulletin 2008/10**

(73) Proprietor: **Joy Medical Devices Corporation**
**Kaohsiung City (TW)**

(72) Inventors:
• **Lu, Pong-Jeu**
**Tainan City 701 (TW)**
• **Lin, Jiin-Huey Chern**
**Winnetka, IL 60093 (US)**
• **Ju, Chien-Ping**
**Kansas City, MS 64137 (US)**

(74) Representative: **Grünecker Patent- und Rechtsanwälte**
**PartG mbB**
**Leopoldstraße 4**
**80802 München (DE)**

(56) References cited:
| | |
|---|---|
| **WO-A1-01/13822** | **WO-A1-98/31292** |
| **US-A- 4 671 263** | **US-A- 5 755 797** |
| **US-A1- 2002 058 947** | **US-A1- 2003 032 964** |
| **US-A1- 2004 196 735** | **US-A1- 2005 222 538** |
| **US-A1- 2005 234 498** | |

**Description**

Field of the Invention

The present invention is related to a technique for delivering an orthopaedic paste into a bone, which will harden in the bone and act as a medical implant. The orthopaedic paste can be any known flowable orthopaedic filling material including; for example, a liquid-powder mixture and a viscous liquid containing a polymeric material.

Background of the Invention

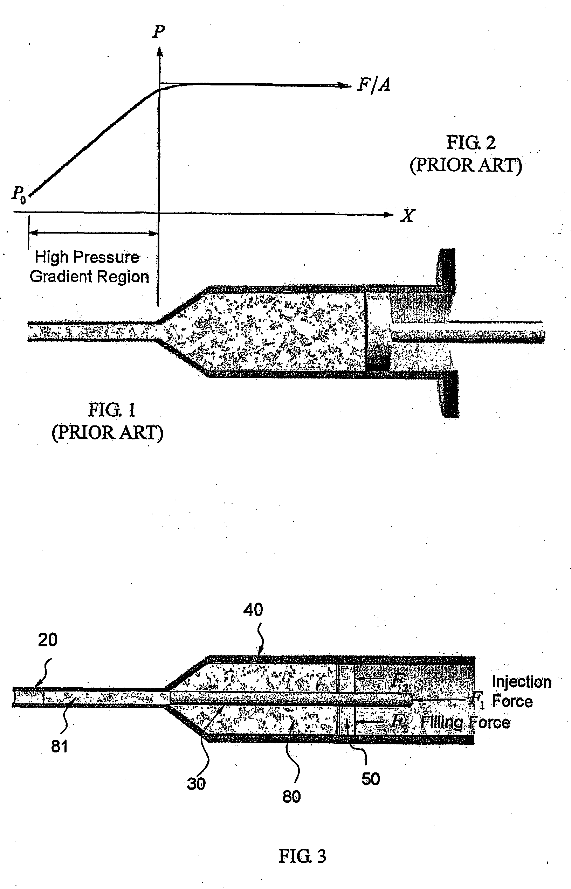

Fig. 1 shows a scheme of a common method of delivering an orthopaedic paste from a container to a designated spot (bone cavity) through a thin tube. In general, the orthopaedic paste, which is a liquid-solid two-phase mixture stored in a container or reservoir, is intended to be transported through a thin tube into a designated spot in a bone structure, tissue or organ of a diseased subject. Conventionally used is a tapered cone-cylinder design which connects the container and the thin tube (syringe), wherein a driving force is applied for orthopaedic paste delivery. For this one-step, direct forced-filling method, the liquid and powder of the orthopaedic paste would tend to separate upon the exertion of the force during the filling process. The underlying physics is explained below.

Generally speaking, the speed of delivery is slow for the method illustrated in Fig. 1. The physical condition occurring in the container throughout the filling process can be described using a static equilibrium concept. The internal pressure developed is approximately equal to the applied force divided by the surface area of the back plate, namely, *F/A.* This pressure (P) is almost uniformly distributed everywhere except around the exit region of the container, i.e. the region near to the thin tube, where the pressure drops drastically to the ambient pressure ($P_0$) of the designated spot, as shown in Fig. 2. This locally developed pressure gradient around the junction of the container and the tube forms the major mechanism that drives the fluid portion of the orthopaedic paste mixture out of the tiny orifice of the container. This can be illustrated by the incompressible Navier-Stokes equation of motion for a fluid flow,

$$\underbrace{\rho \frac{\partial u}{\partial t}}_{unsteady} + \underbrace{\rho\, u \frac{\partial u}{\partial x} + \rho\, v \frac{\partial u}{\partial y}}_{convection} = \underbrace{-\frac{\partial p}{\partial x}}_{\substack{pressure\\gradient}} + \underbrace{\mu\left(\frac{\partial^2 u}{\partial x^2} + \frac{\partial^2 u}{\partial y^2}\right)}_{\substack{viscous\\effects}}$$

In which, *p, u, v, p* and $\mu$ are respectively the density, velocity components, pressure and viscosity of the fluid, and (x, y, t) axe the Cartesian and time coordinates. The orthopaedic paste delivery motion is in general very slow so the unsteady and convection terms can be neglected, resulting in a balance of the pressure gradient and the viscous terms. In other words, the locally developed pressure gradient drives the fluid motion by overcoming the internal or wall friction as the fluid is ejected out. Owing to the large solid-liquid density ratio, the speed of the fluid usually exceeds that of the solid particles, causing the separation of the orthopaedic paste constituents. The fluid part of the orthopaedic paste will continuously flow out of the container because of the continuity characteristic of the flow motion. Nevertheless, the solid part of the orthopaedic paste experiences different physical mechanism as the force is applied. In the beginning, a diluted orthopaedic paste is ejected because more liquid than solid part is compressed out of the container. The remaining orthopaedic paste gets drier during the pressurization period. The small exit area prohibits the dried solid particles from moving quickly out of the container. Except for the initial powder that drifts out of the container, with the carrier liquid, the remainder powder of the orthopaedic paste will be closely packed or interlocked together, resulting in a static equilibrium chunk due to the loss of fluidity, This liquid-solid separation mechanism explains why the one-step, direct forced-filling device often fails as a satisfactory orthopaedic paste delivery injector, especially for a minimally invasive surgical procedure.

WO 98/31292 discloses a method and an apparatus for introducing and compacting bone graft material in an enlarged femoral cavity including a dispenser having a barrel containing bone graft material and a plunger inside the barrel. The barrel is provided with a nozzle for the dispensing of the bone graft by moving an ejector/compactor through the barrel and through the dispensing nozzle.

US patent application publication No. 2005/0113843 A1 discloses an injection system for delivering viscous fluids. Exemplary fluids included flowable hard tissue implant material such as Polymeth,ylmethacrylate (PMMA). The implant material injection system comprises a pressure driver, and a separate container for implant material, wherein said driver and said separate container are adapted to form a sealed pressure-tight interface between each other, said pressure driver comprising a piston and a sleeve, wherein said piston and said sleeve are adapted to draw implant material into at least a portion of a chamber defined by said sleeve upon retracting said piston and to expel implant material from said pressure drive at a pressure level upon advancing said piston. Although this design will work for delivering low viscosity fluid or paste; nevertheless, this design has at least the following disadvantages in delivering highly viscous cement like CPC:

a) The lack of a direct pressure onto paste in the reservoir may result in difficulty in easily moving a highly viscous paste to the "standby" position (the space defined by the "sleeve" in this design).
(b) The use of the sleeve may help pressure build-up within the sleeve to more easily drive a low viscosity paste into the injection tube. However, this sleeve-induced pressure build-up may promote undesired solid-liquid-separation in delivering high viscosity cement. In addition, the presence of the sleeve takes extra effort to operate.
(c) The significant difference in diameter between the sleeve and the injecting tube also promotes the solid-liquid separation of CPC.

US patent application publication No. 2004/0174768 A1 discloses a bone cement mixing and delivery device for mixing a powdered copolymer and a liquid monomer to form a bone cement and delivering the bone cement. The device includes a cartridge having a distal end and a proximal end and defining a mixing chamber between the distal end and the proximal end. A transfer mechanism having a cap and a stem supporting a piston and a plunger is connected to the distal end. The transfer mechanism includes a first advancement mechanism for advancing the piston and plunger in unison and a second advancement mechanism for moving the plunger independent of the piston. The assembly further includes a removable handle having a shaft for attachment of a mixing blade and a quick-release connector and a release button for locking and unlocking the mixing blade from the removable handle. This design has an advantage of complete transfer (injection) of the bone cement without leftover, if the cement is transferable. However, a solid-liquid separation of the CPC cement will occur during the first advancement when this device is used for the reasons recited in the above.

US patent No. 6,033,105 discloses a unitary, fully integrated, bone cement mixing and dispensing system by providing a single housing comprising a mixing chamber integrally combined with a delivery chamber or tube, which eliminates user exposure or handling of the mixed bone cement. In this prior art invention, the two chambers of the integrated system are movable between two alternate positions, a first position wherein each chamber is sealed from the other, and a second position wherein the two chambers in direct communication with each other. In this way the mixing chamber is operated independently from the chamber for preparing the bone cement and, once prepared, is easily transferred from the mixing chamber through the delivery chamber. A dual drive assembly is provided such that it may an independently rotate a mixing blade in a first speed and direction, and independently drive the speed and rotation of a feed controlling element at a second speed and direction. The rate of speed or rotation of the mixing portion is thereby easily controlled to any desired level, relative to the rotational speed of movement of the controlling portion in the delivery chamber. In this prior art design a high viscosity CPC cement cannot be "pulled" into the injection tube without a direct pressure being applied directly on the CPC cement in the mixing chamber. When pressure is applied, the small opening (drastic change in diameter) triggers the solid-liquid separation of the CPC cement. WO 01/13822 A1 describes a tool for injecting bone graft material with the feature of the preamble of claim 1. The tool comprises a small channel with a slotted axis port into which bone graft material can be kept and a plunger within the small channel that can be moved to push the tent bone graft material out of the channel into a filled tube.

Summary of the Invention

A primary object of the present invention is to provide a tool and method for continually delivering an orthopaedic paste stored in a chamber into a bone through a tube.
Another object of the present invention is to provide a tool and method for delivering an orthopaedic paste stored in a chamber into a bone through a tube in the absence of a pressure build-up in the chamber during the delivery.
Still another object of the present invention is to provide a tool and method for delivering an orthopaedic paste comprising particles and liquid into a bone through a tube without significantly changing the solid/liquid ratio of the paste dispensed through the tube.
In order to accomplish the aforesaid objects of the present invention, it is provided an orthopaedic delivery tool with the fatures of claim 1 and a method with the features of claim 9.

Brief Description of the Drawings

Fig. 1 is a schematic partial cross-sectional view of the prior art one-step, direct forced-filling orthopaedic paste tool.

Fig. 2 shows a pressure profile in the paste advance direction (X) of the prior art one-step, direct forced-filling orthopaedic paste tool shown in Fig. 1.

Fig. 3 is a schematic partial cross-sectional view of a horizontal two-step orthopaedic paste feeding device of the present invention.

Fig. 4 is a schematic partial cross-sectional view of a vertical two-step orthopaedic paste feeding device of the present invention.

Fig. 5 is a schematic cross-sectional view of a vertical two-step orthopaedic paste feeding tool of the present invention.

Fig. 6 is a schematic diagram showing the control loop for orthopaedic paste injection used in the tool shown in Fig. 5.

Fig. 7 is a schematic partial cross-sectional view of an orthopaedic paste mixer.

Fig. 8 is a schematic front view of a perforated balloon for entrapping an orthopaedic paste in a bone cavity until the paste is hardened in a bone cavity.

Fig. 9 is a schematic side view of the, perforated balloon shown in Fig. 8.

Fig. 10 is a schematic front view of a surgical tube having a means at a front end thereof for preventing blood/body fluid from entering the surgical tube.

Fig. 11 is a schematic cross-sectional view of the surgical tube shown in Fig. 10. Fig. 12 is schematic side view of a filler for expanding a bone cavity.

Fig. 13 is schematic side view of another filler for expanding a bone cavity.

Figs. 14 to 17 show schematic side views of various stages of an operational process for expanding a bone cavity by using the filler of the present application.

## Detailed Description of the Invention

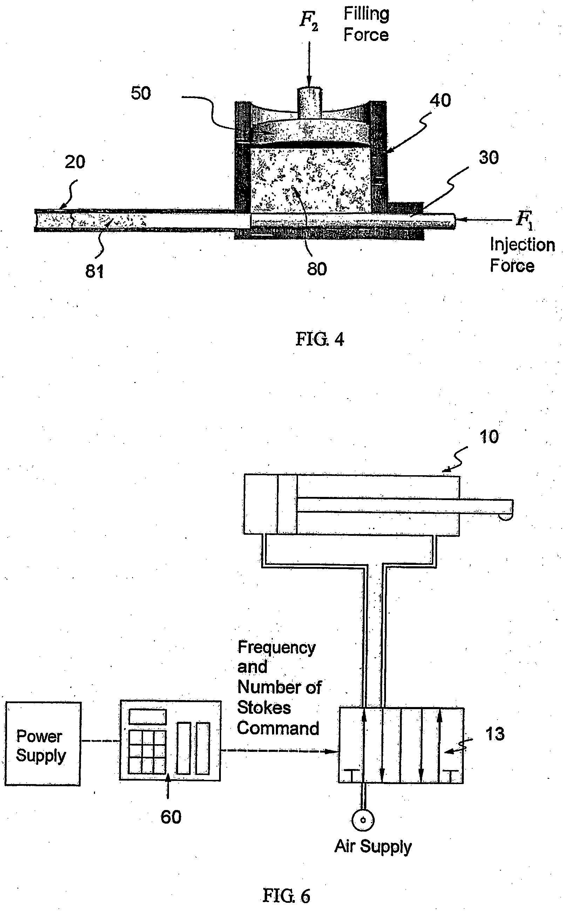

The orthopaedic paste delivery process of the present invention can be facilitated using a novel two-step method described below. Figures. 3 and 4 depict two representative designs, namely, the horizontal and vertical feeding devices [Note: Other angles are also possible]. In both designs a thin injection (outlet) tube 20 is connected to one end of a storage container 40 with a back plate 50 being placed over the other end for pressurization. An injection rod 30 is used for pushing the orthopaedic paste 80 into the outlet tube 20. In the forward stroke motion, an orthopaedic paste segment 81 is pushed into the tube. As the rod 30 is withdrawn, a low-pressure void column will be generated accompanying the rearward stroke motion of the rod 30. The surrounding orthopaedic paste will immediately fill this void column space due to a "vacuum suction" effect as well as the applied back pressure. Unlike the conventional one-step design shown in Fig. 1, the present applied filling force ($F_2$) or the pressure gradient developed is usually small, which is required only for pressing the orthopaedic paste into the void space created by the previous back stroke. Separation of the orthopaedic paste injection and feeding into different steps with small applied injection and filing forces ($F_1$, $F_2$) characterizes the present design philosophy. The orthopaedic paste feeding step follows the injection step, and the assigned delivery mission is accomplished by an accumulation of the delivered orthopaedic paste segments 81. In both steps the aforementioned high pressure gradient phenomenon would not occur and these two steps can be repeatedly operated. There is literally no limitation on the amount of the orthopaedic paste to be delivered. Moreover, the force applied on the injection rod can be adjusted to overcome the resisting pressure exerted on the outlet end of the tube.

Contrary to the device depicted in Fig. 1, the container shape for the two-step method is not critical. In principle, any shape and orientation of the container 40 can be used. This is due to the advantage generated by the present design since, for any container shape, the applied force can easily feed the orthopaedic paste into the void space created by the previous injection stroke partly due to the aforementioned vacuum suction effect.

The two-step orthopaedic paste delivery system of the present invention consists essentially of four structural components and two external forcing mechanisms. The function of each constituent part is described in the following with reference to Fig. 4.

### 1. Orthopaedic Paste Storage Container 40

The container is used to store the orthopaedic paste to be delivered. The inner wall can be a cylinder of any cross sectional shapes. Surface roughness of the inner wall should be minimized to reduce wall friction. Non-stick coating may also be used to further enhance the feeding effectiveness.

### 2. Injection (outlet) Tube 20

This tube is preferably a substantially constant cross sectional tube, which can be either flexible or rigid. Orthopaedic paste segments will be delivered through this tube into the designated delivery spot.

### 3. Injection Rod 30

This injection rod has substantially the same cross sectional shape (or a modified shape) as the injection tube 20. The length of the injection rod is selected in such a way that, in the forward stroke, the rod end barely touches the entrance plane of the injection tube 20. For this motion mode, the diameter of the injection rod can be larger than the inner diameter of the injection tube. Practically, the injection rod should have a diameter not too larger than the inner diameter of the injection tube. Preferably, the injection rod should not have a diameter larger than the inner diameter of the injection tube by five times; and more preferably, the injection rod should not have a diameter larger than the inner diameter of the injection tube by three times. For another motion mode, the injection rod may be partly inserted into the injection tube. In this case, the diameter of the injection rod should be a little smaller than the inner diameter of the injection tube. Clearance can be properly selected to facilitate the stroke motion of the injection rod. The length of the rod can vary, depending on how much orthopaedic paste is intended to be left in the injection tube beyond the designated delivery spot.

### 4. Back Plate 50

Back plate is used for transmitting the pressure required to feed the orthopaedic paste into the void column created by the previous back stroke as well as to prevent the spillage of the orthopaedic paste during the feeding process.

### 5. Orthopaedic Paste Delivery Mechanism (not shown in the drawings) for providing the injection force $F_1$

This mechanism can be any manually, pneumatically, hydraulically, or electro-magnetically driven devices. The force $F_1$ applied should be greater than or equal to the sum of the resisting load occurring at the delivery spot plus the friction force exerted by the outlet tube wall. Control can be applied to facilitate the reciprocal motion of the injection rod connected to this forcing mechanism.

### 6. Orthopaedic Paste Feeding Mechanism (not shown in the drawings) for providing the feeding force $F_2$

This mechanism can be any manually, pneumatically, hydraulically, or electro-magnetically driven devices. The applied force $F_2$ should be adjusted barely enough to feed but not overly compress the orthopaedic paste contained inside the container. The feeding is largely assisted by a vacuum suction effect induced by the rearward stroke of the injection rod, which creates a low-pressure void column space.

In view of the above, the present invention has at least the following major features:

1. The separation of orthopaedic paste delivery into two uncorrelated (independent) steps of feeding and injection.
2. Orthopaedic paste injection is accomplished without a locally developed high pressure gradient.
3. Orthopaedic paste feeding is accomplished by filling the low-pressure void column space assisted by a vacuum suction effect which is a separate (irrelevant) motion to the orthopaedic paste injection
4. Total orthopaedic paste delivery is accomplished by a series of reciprocal injection and feeding strokes rather than the one-step, direct forced-filling process.

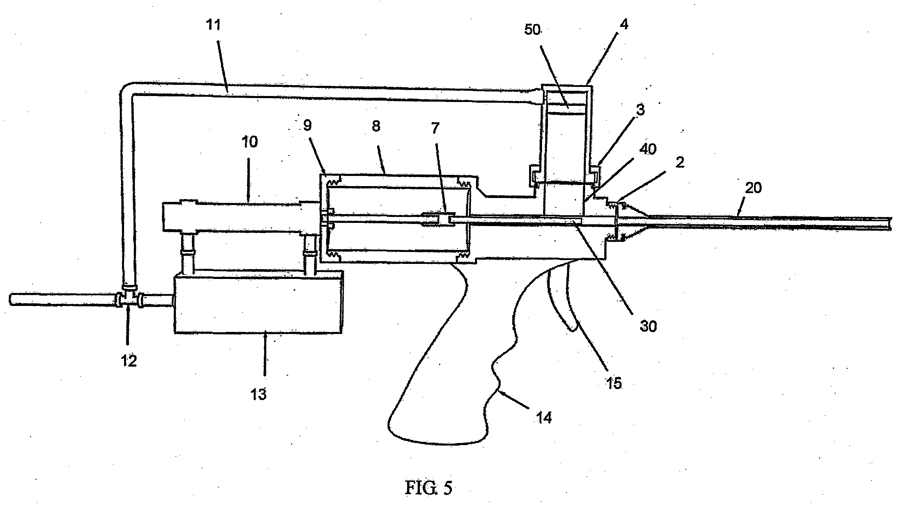

An orthopaedic paste delivery tool constructed according to one of the preferred embodiments of the present invention is described in the following with reference to Figs. 5 and 6.

The orthopaedic paste delivery tool depicted in Fig. 5 mainly consists of four modules: 1) Injection Tube; 2) Main Body; 3) Pneumatic Driver; and 4) Controller. For each module the function and components included are described as follows.

### 1. Injection Tube 20

This tube 20 is the access channel for the orthopaedic paste to be delivered to the designated place in the diseased subjects. The diameter of the tube 20 is around 1-3 mm, preferably 2-3 mm, and the material adopted could be stainless steel or other metal or polymers. One end of this tube is attached to the main body module via a quick connector 2. This injection tube 20 could be disposable after orthopaedic paste delivery. Quick connector 2 eases the attachment and detachment of the tube 20 to and from the main body module. Upon finishing orthopaedic paste delivery, by unscrewing the quick connector 2, the injection tube 20 can be detached from the rest heavy modules for orthopaedic paste solidification. A pushing rod can be inserted from behind the tube end to further add the remaining orthopaedic paste in the tube to the cavity.

2. Main Body Module

This module connects respectively the injection tube 20, feeding cup 4 and the pneumatic driver module. This module is secured on a handle 14 for the operator to easily accomplish the orthopaedic paste delivery mission. Orthopaedic paste is stored in advance in the feeding cup 4 which is mounted onto and off a storage container 40 provided on the main body via a quick connector 3. At the base of the feeding cup lie a back plate 50 and a through hole connected to the pneumatic line 11. Pressurized air is supplied through the pneumatic line 11 into the feeding cup 4. The back plate 50 then works as the interface to exert pressure on the orthopaedic paste for feeding. Sealing is applied around the rim of the back plate 50 as well as at the quick connector 3 base so as to maintain the feeding pressure and keep the air from leakage. An orthopaedic paste injection rod 30, as propelled by the pneumatic driver, works back-and-forth at the bottom of the container 40 as a driving force to push the orthopaedic paste segment into the injection tube 20. The length of the injector rod 30 is selected in such a way that, in the forward stroke, the rod end barely touches the entrance plane of the injection tube 20. This design length can avoid the back spillage of the orthopaedic paste in the injection tube as the injector is withdrawn in the backward stroke. In order to facilitate orthopaedic paste feeding, the inner wall of the feeding cup 4 can be properly configured and/or coated with non-sticking materials.

3. Pneumatic Driver Module

This module uses a pneumatic cylinder 10 and directional control valve 13 which are commercially available components. Since in general the size of the stroke rod of the pneumatic cylinder 10 is different from that of the injection rod 30, an adaptor 7 is used to connect together the pneumatic cylinder 10 and the injection rod 30, allowing power transmitted from the air supply to the delivered orthopaedic paste. The stroke distance determines the model type of the pneumatic cylinder selected. In order to fix the pneumatic cylinder 10 and the directional control valve 13 firmly onto the main body, a connector 8 and connector cap 9 are used. Note that the connections at the two ends of this connector structure should be air tight so as to assure the feeding pressure desired. Pressurized air enters the pneumatic cylinder 10 as well as the feeding cup 4 through a two-way connector 12. This pressure supply can be adjusted in accordance with the resisting pressure at the cavity where orthopaedic paste is intended to be placed. The directional control valve 13 is an electromagnetic valve which can be used to control the air ways inside the pneumatic cylinder 10. As the air way is selected by the electromagnetic circuit, the rod of the pneumatic cylinder will be actuated in either forward or backward movement.

4. Controller 60

The controller layout is illustrated in Fig. 6. The controller 60 is a circuit board which is designed according to the operational requirements. Presently the control parameters are the stroke frequency and the total number of strokes. The control command can be sent to the directional control valve 13 via an electric circuit together with an ON/OFF trigger 15 mounted on the handle 14. As the trigger 15 is pressed down, the control command will be activated to drive the injection rod 30 moving back-and-forth with a preset frequency and total number of strokes. The feeding motion can be stopped as required when the trigger 15 is released to spring back and the control command gets cut off. The air ways regulated by the directional control valve 13 is also sketched in Fig. 6. The left air way is a route for pushing the injector rod forward and vice versa for the right air way. The amount of orthopaedic paste to be delivered can be calibrated and decided by the specified number of strokes. The feeding frequency, however, is determined by the resisting pressure and the orthopaedic paste setting time. Generally, the higher the resisting pressure, the slower the feeding frequency because the time required for the exertion of force to move the injector rod will increase. For a fast-setting orthopaedic paste the feeding frequency should be higher and the air supply pressure should be raised accordingly.

Orthopedic Paste Mixer

A miniature, motor-driven, piston-type (with a sealed sliding head) impeller-induced mixing apparatus is designed for mixing orthopedic cement for orthopedic or dental uses. The mixer consists of a container with a specially designed piston, a miniature electric motor and clutch, and an impeller housed in a base connected with an air supply system. These three components can be assembled using convenient screws or quick connectors, allowing fast assembly and detachment. The sealed, sliding piston head, which is allowed to move up/down before, during and/or after mixing, is a special design which enables easy de-airing and other functions to be accomplished.

One of the preferred mixing modes that can be applied to the mixing system is mixing under pressurized condition (cushion-air mixing). That is the mixing is carried out beneath a cushion-air layer. In so doing, prior to mixing, a cushion-air layer is reserved inside the container on top of the cement powder and setting solution. One function of the cushion air pressure is to minimize the gasification of the solution, resulting in the delay of solidification (increasing working/setting time of the cement).

The mixing process is accomplished using impeller-induced vortices generated by a high-speed rotational stirring. Upon finishing mixing, the mixer is inverted to make the mixed cement paste drop back into the container. The cushion air layer, as propelled again by the impellor, becomes a cement scrubber, in additional to the gravitational and centrifugal effects, to further sweep off the cement remainder stuck on the impellor and the base wall, enabling a more complete collection of the cement mixture for subsequent uses (e.g., injection) in the delivery process.

For an orthopedic surgery (e.g., a minimally invasive spinal surgery) involving injection of an orthopedic cement paste into a bone cavity through a thin tube, appropriate amounts of the cement powder and setting solution with an appropriate powder/liquid ratio are mixed prior to injection. The mixing can be conducted ether manually by hand or automatically using a mixer. The powder/liquid mixture (paste) is quickly transported into a reservoir/container for injection.

It is almost unavoidable to have certain amount of air trapped into the paste during mixing. The trapped air can act as voids and largely affect the mechanical performance of the cement after being set. To reduce the trapped air effect, the method of vacuum mixing has often been used or proposed for use. However, since many setting solutions comprise highly volatile liquid components, the powder/liquid ratio can increase during mixing and/or transportation of the paste from the mixer to the reservoir, if the processes are not carefully controlled. In this case, vacuum mixing can speed up the volatilization of the liquid component, resulting in further increase in the powder/liquid ratio of the paste, which not only causes the paste to become drier and harder to inject, but also changes the properties of the cement.

The aforementioned problem of the change in powder/liquid ratio during mixing and/or transportation of the cement paste can be largely solved by applying the present mixer design. The design allows cushion-air (pressurized) mixing. It also minimizes the exposure of the cement paste to the outsider environment during mixing as well as transportation of the paste. Furthermore, the new design allows the mixer to be quickly and easily attached to the reservoir (storage chamber) of any compatible cement delivery system ready for injection.

For the present cement paste preparation, care must be exercised to avoid air be trapped in during mixing. In a mixer container, air pre-exists either in the solid powders or in the space besides those occupied by the powder and setting solution. Cushion-air mixing, retains a layer of air on top of the solid powder and setting solution prior to mixing. When mixing starts, except for the air already trapped in the solid powder, the chance for air to come into cement paste originates in the engulfing motion occurring at the air-fluid interfaces. Air layer will be imposed with high pressure provided by air supply system in the cushion-air mixing. This pressurized air layer can be more resistant to the air-fluid interface break-up, hence making the interface more planar during mixing to discourage the phenomenon of air entrainment.

The present mixer is a miniature and light-weight device so it can be flipped over easily. The mixer apparatus can be inverted up-side-down when mixing is finished. To ease the transport of cement back to the container, a sufficient space is created between the cement paste and the piston head. Gravitation will make most cement paste fall into the container, with the remainder stuck on the mixer blades and the base wall as well. Turn on the motor switch again will cause mixer blades rotate with high-speed in the air layer. The agitated air layer will work as a scrubber that blows off the cement paste on the base wall. The centrifugal force, however, will clean up the paste attached on the blades. The inverted mixer hence has most its mixed cement paste collected in the container. The container can be unscrewed and moved to be installed on any compatible cement delivery system for subsequent uses (e.g., injection).

The cement mixing and transfer processes are conducted in a closed container with minimum exposure to outside environment. As mentioned earlier, this can reduce evaporation of the liquid (especially highly volatile liquids) during the preparation (mixing) and transportation of the paste.

Pressurized mixing chamber condition allows mixing process to be conducted in a longer time and hence a more thorough fashion. Moreover, the impeller preferably consists of fours blades, and more preferably a cascade of a lower two-blade bar and an upper two-blade bar, with the adjacent blades having opposite dihedral and twist angles, to generate a vortex-induced mixing, which directly provides a high-gradient shear flow field to promote much effective molecular-level mixing, namely momentum and mass transfer, to occur.

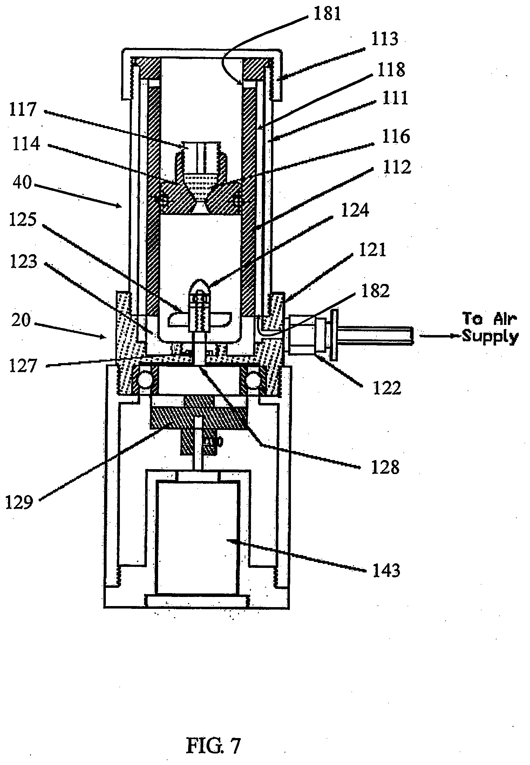

There are three basic components that make up the present device, as shown in Fig. 7. The mixer container 140 situates on top of the apparatus. This container 140 can be used as the feeding cap 4 of the delivery tool shown in Fig 5. A low friction (to cement) material, such as Teflon or porcelain-coated material, is used for inner sleeve 112 for non-sticking purpose. Between the Teflon sleeve 112 and the outer container wall 111 are slot-shaped hidden channels 118 manufactured for pressurized air to come in. An air-sealed piston 114 is inserted for imparting pressure to the cement mixture. A through hole which is drilled on the piston center body with rubber seal (needle seal stub) 116 attached to the end thereof by a hollow fastener 117 is used to fulfill the de-airing requirement.

The mixing mechanism consists of an impeller 124 with a shaft 128 which is rotatably received in a hole of a bowl 120 and sealed using an o-ring 127, and four blades 125 fixed to the upper end of the shaft. The blades 125 are in the bowl 120. The shaft is further connected to a clutch 129 at the lower end for power transmission under the bowl 120. Energy will be transmitted into cement mixture by stirring the mixture using the impeller. The induced vortices serve as the mixing mechanism whose performance depends primarily on the impeller blade configuration selected. The bowl 120 has a Teflon sleeve 123 embedded in a metal housing 121. An annular channel is formed in the bowl 120 between the sleeve 123 and the housing 121, which communicates an air port 122 provided on the housing 121 to an outlet 182 of the channel 118 of the container 140, thereby allowing pressurized air coming from an air supply connected to the air port 122 through the annular channel of the bowl 120, the outlet 182, the channel 118 and an inlet 181 of the channel 118 into the container 140.

An electric motor 134 is located at the bottom of the mixer with sufficient power and torque to drive the impeller 124 through the clutch 129 connected on the motor shaft for power transmission.

The steps of the cushion-air mixing are as follows:

*Step 1*: Remove the cap 113 and piston 114 away from the container 140 and screw this container onto the bowl 120. Pour cement powder and setting solution into the container 140.

*Step 2:* Insert a syringe needle through the needle seal stub 116 for de-airing purpose. Slide and push this piston-needle assembly into the container 140. Air will be squeezed out from the needle when piston 114 moves downward. Leave appropriate volume (e.g., 3-5 c.c.) of air above the liquid surface by adjusting the piston position. *Step 3:* Remove the de-airing syringe needle and screw on the container cap 113.

*Step 4:* Send pressurized air via internal channel 118 to compress the piston 114 and hence raise the pressure in the container 140 to a predetermined value.

*Step 5*: Turn on the motor 134 to stir the powder and liquid contained in the mixer until the cement paste is completely mixed therein.

*Step 6*: Invert (and shake) the mixer and let the mixed cement paste fall into the container 140.

*Step 7*: Turn on the motor 134 again to scrub the cement remainder left on the impeller 124 and base wall into the container 140.

*Step 8:* Unscrew the container 140 off the bowl 120 and mount the cement paste-filled container 140 onto a compatible cement delivery gun for injection.

Method and Device for Forming a Hardened Cement in a Bone Cavity

During an orthopedic surgery (e.g., a minimally invasive spinal surgery) involving injecting a solid-liquid mixed orthopedic cement paste into a bone cavity through a thin surgical tube, a number of problems can occur. Since conventionally-used surgical tubes (guiding tubes, injection tubes, etc.) for such application are open-end tubes, the blood/body fluid of the patient backflows and fills the tube immediately after the tube is inserted into the patient's body (e.g., a collapsed vertebral bone structure) through surgery.

As the orthopedic cement paste is subsequently injected into the blood/body fluid-filled tube, the paste can be diluted and/or dispersed upon contact with the blood/body fluid. The dilution of the paste accompanied with a decrease in solid/liquid ratio (which should remain constant to guarantee the properties of the cement) can largely change the mechanical properties (e.g., strength) of the cement. Furthermore, the warm (body temperature) blood/body fluid can largely shorten the working time of the paste into a surgically unacceptable range. Yet the worst case would be immediate dispersion/disintegration of the cement paste upon contact with the blood/body fluid. This often-observed dispersion/disintegration occurs due to the lack of a reasonable initial strength quickly developed in the paste after mixing of the solid and the liquid.

Various orthopedic cements have been used as implant or filling material in dental and bone prosthesis. The conventional method of forming a set/hardened bone cement in bone cavity involves directly injecting a cement paste into bone cavity, which suffers the followings drawbacks among others:

(i) While the liquid-powder ratio of the cement paste is too high, the strength of the hardened cement becomes too low, that can cause the cement to more easily disperse/disintegrate;

(ii) While the liquid-powder ratio of the cement paste is too low, the viscosity of the paste becomes too high, the working and setting times become too short, and the paste is hard to inject through a syringe;

(iii) Dispersed cement particles in body fluid/blood, especially before being fully set, can penetrate into surrounding tissue that can cause serious hazard during or after surgery.

In the following a method and a device for forming a hardened cement in a bone cavity is disclosed.

More specifically, a method and a device are provided for forming a balloon containing the cement in a bone cavity. The balloon can be dilated by filling the cement paste into the balloon and fractured as desired after the cement is hardened.

More specifically, the balloon can be ruptured in a predetermined (designed) manner/pattern after the cement is hardened.

Preferably the body of the balloon comprises a leaking mechanism for allowing liquid contained in the paste inside the balloon to be expelled from the balloon under pressure.

The present version solves the aforesaid prior art drawbacks because the cement paste sets within the balloon without directly contacting body fluid/blood, and pressure can be developed within the balloon, which expels a portion of the liquid/setting solution contained in the paste out of the balloon and largely increases the strength of the cement, reduces the risk of cement dispersion/disintegration, and also avoids cement paste leaking into the surrounding tissue.

Further, the present version has an advantage of being easy to keep a powder/liquid ratio of the cement paste accurate by monitoring the pressure build-up within the balloon that is important to cement properties such as setting time and strength.

Most of all, the inventive balloon can be ruptured in a predetermined (designed) manner (pattern) after the cement is hardened so that the risk for some random fractured pieces of the balloon to be left in the bone cavity during surgery is minimized.

The central concept of the present version -perforation array- may also be applied for "balloon-less" cement delivery systems. More specifically, the perforation array concept can be used for a device for preventing the blood/body fluid of a patient from entering a surgical tube (guiding tube, injection tube, etc.) before an orthopedic cement paste is injected into a bone cavity through the injection tube.

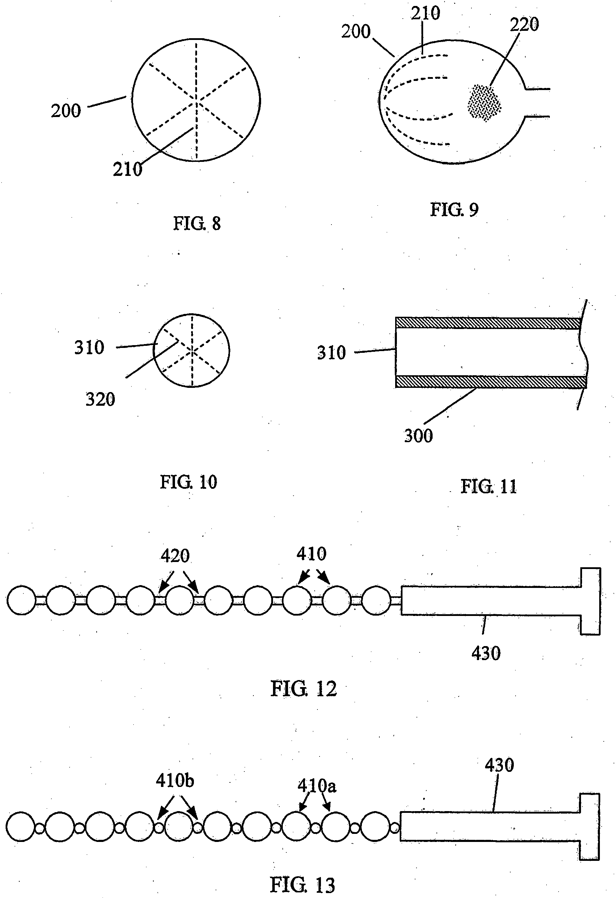

Balloon design (Figs. 8 and 9)

In order for the balloon to be ruptured in a predetermined (designed) manner (pattern) after the cement is hardened, "perforation array" is designed, for example the perforation array 210 shown in Figs. 8 and 9. The perforation array 210 is used mainly to rupture the inflated balloon 200 with predetermined lines/pattern of breakup, although permeability effect is also provided therein when the pore size is carefully controlled. The perforation array is also designed to keep the entire ruptured balloon to remain attached to the injection tube end after being ruptured. Without this design, it is highly likely that some random pieces of the ruptured balloon are detached from the balloon and left permanently in the bone cavity. Ideally the entire balloon should remain attached to the injection tube after being ruptured and can be entirely withdrawn along with the tube.

The perforation array 210 comprises designed patterns of pores, dents, notches, grooves, cuts, etc. and are made on the surface of at least a portion of the balloon. Such pores, dents, notches, grooves, cuts, etc. can be made by any conventional methods. Preferably, these pores, dents, notches, grooves, cuts, etc. are made at or near the central part of the balloon. Preferably, the "lines of perforation" converge around the apex of the balloon, creating relatively weakened spots where rupturing crack would initiate.

Such parameters as pore size, population, spacing between perforations, number of perforation array, and the array size are to be controlled and optimized to result in a required structural characteristics of the balloon.

Although permeability (draining) effect is provided in the design of the perforation array, in order to more effectively drain water and air out of the balloon as the cement paste is injected to fill the bone cavity, micro-pores 220 can be further incorporated over the surface of the balloon 200. The micro-pores can be made by any conventional methods such as laser-drilling or other mechanical methods. These micro-pores can be distributed randomly or in a designed manner/pattern and will be progressively enlarged as cement mixture is continually delivered into the balloon.

Preferably, the balloon is made of an elastic polymeric material (elastomer) with its zero-stress size small enough to be stored conveniently within the cement injector tube. The balloon should have appropriate elastic property which allows the balloon to expand at least 3 times with the cement paste inside the balloon under pressure. The balloon thickness should be selected with sufficient strength when expanding to its intended dilated volume. This dilated balloon should develop appropriate tension to facilitate its rupture and shrinkage. However, the developed tension should not be too large so as to avoid premature balloon rupture. The balloon can be manufactured using, for example, the techniques available in the design of intra-vascular balloon catheter devices, the technique of solution casting of polyurethane or other polymers, etc.

As cement mixture is delivered to fill the bone cavity and expand in dimensions, the balloon surface will confine the cement, with a portion of air and water compelled out of the balloon through aforementioned numerous tiny pores and perforations that have been incorporated in during the manufacturing process. This bone cement filling process can be considered as a static force equilibrium problem. Basically there are three pressures that are involved. Firstly, the bone pressure which is the pressure within the collapsed spine. Without the compression of the body weight, bone pressure equals approximately to the atmosphere pressure.

Secondly, the pressure inside the balloon, or the so-called cavity pressure, which is decided by the bone pressure and the surface in-plane tensile stress as the membrane is inflated to deform. The stronger the balloon material, the larger the bone and cavity pressure differential will result. This pressure differential holds a central role in squeezing the air and/or water out of the balloon. Therefore, in designing the balloon module, a choice of proper elastomer strength, together with the predetermined pore size and porosity, may decide how effectively the cement mixture will be dehydrated.

Thirdly, the delivery pressure which is provided by the pressure source of the cement delivery system. Usually the delivery and cavity pressures are equal as long as the seeping speed of water through the tiny pore channels is low.

As balloon is inflated against the collapsed spine, the in-plane membrane stress will develop as the balloon swells. This membrane stress may produce a higher interior pressure for the balloon, which is the major mechanism that drives air and liquid (with much smaller molecular weights and sizes as compared to those of the cement) out of the cavity space while retaining primarily the solid part of the cement within the sac of the balloon. The dehydration is naturally combined with the cement delivery process and the flexible balloon material will help the cement filling conformal with the shape of the enlarged interior bone space.

While the inside space of the balloon is tightly filled with cement paste which supports the balloon morphology, a proper balloon size is selected prior to the start of cement injection For different patients or cases, an assessment of how much cement should be delivered ought to be decided in advance using appropriate instruments such as X-ray, etc. As the selected balloon is inflated to its designed volume, the membrane stress magnitude should be sufficient enough to hold the cement in place during injection and dehydration, yet maintaining an appropriate safety margin to the rupture of the balloon. This stretched state would result in a provision for an easy balloon rupture as additional force is applied later to extract the balloon out of the bone cavity.

A series of balloon sizes should be used for different cement volumes to be delivered. These balloons differ basically in lengths and thicknesses since the lateral dimensions are restricted to be the same so as to enable the balloons stored within the distal end of the cement delivery tube. The tube size (preferably smaller than 5 mm and more preferably smaller than 3 mm in diameter) and the final expanded cavity size will determine the strain required for the balloon material. This strain, in turn, will determine which operational elasticity range the balloon should possess. Furthermore, as an elastomer is selected to allow for the required balloon stretching, the perforation size, location and porosity should be carefully examined so as to make the balloon rupture, varying according to different balloon sizes, easier in the last extraction step.

The dilated balloon will be ruptured and shrunk back to its original zero-stress state. This rupturing constitutes automatically an extraction function of the balloon. The higher the tension in the balloon, the more effective is the rupture and back extraction of the balloon. However, care must be exercised to avoid excessive tension developed which may promote undesired premature balloon rupture caused by contacting with the rough surface of the bone cavity during its cement delivery period.

The pressurization of the delivered cement has two major functions that characterize the present method. The first is the function of expanding the collapsed bone structure to some desired shape and size. Through the fluid motion of the cement paste, pressure can be transmitted to make the cement fill tightly within the bone cavity and at the same time push the bone structure restoring back to its original shape and size. Secondly, during the pressurization of the cement, a portion of air and liquid/setting solution content of the cement can be squeezed out of the balloon. Pressurization and liquid extraction will help the solidification of the cement, which is critical for the structure and strength development as the cement is hardened within the balloon.

The Cement delivery procedures are described as follows:

*Step 1*

Drill a through hole on the diseased spine to create a cylindrical cavity. Estimate the volume of the cement to be delivered. Before delivering an appropriate amount of cement paste into a bone cavity using a tube and an injector plug such as the outlet tube 20 and the injection rod 30 shown in Fig. 5, the balloon is connected to the injection end of the injection (preferably cylindrical) tube by a mounting mechanism, for example, having an annular groove provided on an outer surface of the tube, and a ring adapted to elastically grip the annular groove. The injection end of the cylindrical tube is inserted into an opening of the balloon, so that a neck of the balloon covers the annular groove; and putting the ring, which is a closed ring or a C-shaped ring, on the neck of the balloon and clamping it at the annular groove on the tube. The mounting mechanism including the annular groove and the ring is similar to that described in US patent application publication No. 2004/0186481 A1.

*Step 2*

Expand the balloon by pushing the injector plug to compress the cement paste with a pre-calibrated pressure (preferably about 1-5000 psi, and more preferably 10 - 1000 psi) until the damaged bone is expanded to the desired shape and size, wherein a portion of liquid and air are expelled from the cement paste via the draining mechanism. Water/air drainage occurs only significantly in the later stage of cement delivery as the in-plane stress is developed in the membrane which creates the pressure differential to compels the water/air out.

*Step 3*

Fine adjust the expanded cavity size (Note: Fine adjustment may be required to account for the volume loss due to water drainage and cement spillage. X-ray or other monitoring facility may be used for monitoring the process). Hold the injector rod with appropriate back pressure.

Pressurize the cement paste for a period of time until the cement is hardened in the balloon.

*Step 4*

Extract the injector tube as well as the balloon outward (with the injector rod held fixed) and initiate the balloon rupture originating from its apex along the designed perforation lines. Extract the ruptured balloon while holding the injector plug against the cavity outlet until the balloon clears the exit. Withdraw the whole delivery system out of the patient body.

Device and Method for Preventing Liquid from Entering a Surgical Tube

During an orthopedic surgery (e.g., a minimally invasive spinal surgery) involving injecting a solid-liquid mixed orthopedic cement paste into a bone cavity through a thin surgical tube, a number of problems can occur. Since conventionally-used surgical tubes (guiding tubes, injection tubes, etc.) for such application are open-end tubes, the blood/body fluid of the patient backflows and fills the tube immediately after the tube is inserted into the patient's body (e.g., a collapsed vertebral bone structure) through surgery. As the orthopedic cement paste is subsequently injected into the blood/body fluid-filled tube, the paste can be diluted and/or dispersed upon contact with the blood/body fluid. The dilution of the paste accompanied with a decrease in solid/liquid ratio (which should remain constant to guarantee the properties of the cement) can largely change the mechanical and other properties (e.g., mechanical strength) of the cement. Furthermore, the warm (body temperature) blood/body fluid can largely shorten the working time of the paste into a surgically unacceptable range. Yet the worst case would be immediate dispersion/disintegration of the cement paste upon contact with the blood/body fluid. This often-observed dispersion/disintegration occurs due to the lack of a reasonable initial strength quickly developed in the paste after mixing of the solid and the liquid.

A device and method for preventing a liquid from entering a tube is disclosed in the followign. More specifically, the device and method disclosed are for preventing the blood/body fluid of a patient from entering a surgical tube (guiding tube, injection tube, etc.) before an orthopedic cement paste is injected into a bone cavity through the injection tube.

The above-mentioned drawbacks can be greatly overcome by applying the present device at or near the front (leading) end of the tube that prevents blood/body fluid of the patient from entering the tube, yet allow the cement paste to pass through (break) the device during injection of the paste.

As shown in Figs. 10 and 11, the device 310 can be a water-resistant polymeric film, the strength of which (depending on such factors as inherent strength of the material used to make the film, the thickness of the film, etc.) is adjusted to be high enough to withstand the pressure exerted internally from the blood/body fluid of the patient, yet weak enough to allow the injected paste to easily break the film and enter the bone cavity. The film can be prepared from any conventional polymeric material and by any conventional method. The film can be attached to a tube end of a surgical tube 300 by any conventional method.

Preferably, patterns 320 of dents, notches, grooves, cuts, etc. are made on the surface of at least a portion of the film. Preferably, the dents, notches, grooves, cuts, etc. are made at or near the central part of the film. These dents, notches, grooves, cuts, etc. are designed to assist the breaking process of the film by the injected paste and to keep the fractured film still attached to the tube end (rim/edge) after being broken. Without this design, the fractured film can fall apart into pieces and be mixed into the paste entering the bone cavity. Ideally the entire film should remain attached to the tube after being fractured and can be entirely withdrawn along with the tube after injection.

The film can further have multiple micropores (tiny holes/perforations) in it. These tiny holes are small enough to prevent water (blood/body fluid) from passing them through, yet large enough to allow air to pass through. The design of such micropores/tiny holes, which are penetrable to air but impenetrable to water, prevents air pressure built up within the tube (front end) during injection of the cement paste. The build-up of air pressure in the tube (in front of the injected paste) can cause the injection to become difficult (being resistant), or even cause the film to fracture prematurely (before the injected paste reaches the film). The presence of the liquid-impenetrable film also helps maintain a constant powder/liquid ratio in the paste. As mentioned earlier, a constant powder/liquid ratio is critical to the properties of the cement. The micropores can be prepared by any conventional methods.

Preferably, the film is prepared from a biocompatible polymeric material. More preferably, the film is prepared from a biocompatible and biodegradable polymeric material. This is based on the consideration that, in case some fragments/pieces of the film break off and are carried into the bone cavity, a biocompatible and biodegradable material would be safer than other materials. Many existing biocompatible and biodegradable polymers can be selected.

The device 310 can also be a one-way lid /valve attached to the front end of the tube 300. This one-way lid prevents the blood/body fluid from entering the tube, while allowing the injected paste to open and pass through the lid entering the bone cavity. The lid can be made from a metal or polymer and can be attached to the tube end by any conventional methods.

Device and Method for Expanding a Bone Cavity

Treating a patient with a collapsed bone structure (e.g., compression fracture in vertebral body) often involves first recovering (expanding) the collapsed bone structure to its original structure (shape and size). Following it an orthopedic cement paste is injected into the expanded (and often porous) bone cavity. The hardened/cured cement provides additional strength to the bone structure being healed.

Conventionally-used is a polymeric balloon which can be expanded by pressurizing a liquid (e.g., water) into it after being inserted into the collapsed bone structure. After reaching a desired size, the liquid is drained out and the balloon is withdrawn from the cavity. The expanded cavity is then injected with orthopedic cement paste.

One inherent problem with the polymeric balloon for this treatment is the relatively low hardness and strength of the balloon material, such as commonly-used PU material. Occasionally during its expanding process, the expanded polymeric balloon breaks open as a result of its impingement upon surrounding irregularly-shaped bone fragments. Another potential problem with the balloon method is that the "soft" balloon can occasionally be "arrested" (entangled) by bone fragments which can interfere with the withdrawal of the balloon from the bone cavity.

The aforementioned problems with the balloon method can be largely overcome by applying the present device/method for expanding the collapsed bone structure, as described below.

The present device comprises a highly flexible cavity filler which can be easily inserted in and withdrawn from the bone cavity. Unlike the balloon concept, the filler is not inflated (dilated) or filled with liquid during the expansion process, which effectively overcomes the aforementioned problems with the balloon method. The level (degree) of expansion of the bone cavity is controlled by the volume of the filler inserted into the bone cavity.

The filler can be made from a highly elastic wire. The wire filler can be made from polymer or metal with a diameter from about 0.1 mm to about 5 mm, preferably from about 1 mm to about 3 mm. Due to its highly flexible nature, the wire can easily deflect in the bone cavity while impinging against the bone cavity wall. With increasing the volume (or length) of the wire inserted, the bone cavity is expanded and "filled" with the twisted (deflected) wire. The insertion of the wire can be conducted through a surgical tube and assisted by applying a pressure onto the wire. In so doing the wire can be attached onto a stud (or rod) and then pushing the stud into the tube. When a desired cavity volume is reached, the stud along with its attached wire is withdrawn from the tube.

The filler can also be prepared by linking multiple objects, such as beads, into a highly deflectable "necklace." The beads can be made from metal or polymer, can be rigid or flexible, and can be linked together using any conventional method such as that used for making a necklace.

The beads can be of any shape, but preferably of spherical shape to reduce friction during insertion and withdrawal of the filler. As shown in Fig. 12, spherical beads 410 of same size are linked by a wire 420, wherein one of the wire 420 is connected to a stud 430. The beads 410 and wire 420 constitute the filler, and the stud 430 works as a filler holder. Beads of different sizes can be combined to form the "necklace." For example, larger beads 410a and smaller beads 410b can be linked alternately (a smaller bead can be placed between every two larger beads) as shown in Fig. 13.

Each individual bead has a diameter from about 0.1 mm to about 5 mm, preferably from about 1 mm to about 3 mm. The distance between two adjacent beads should not be too large to prevent entangling of the beads, which may inhibit the beads from being withdrawn from the tube. Specifically, the distance between two adjacent beads should be less than the diameter of the beads, preferably less than one half of the diameter of the beads.

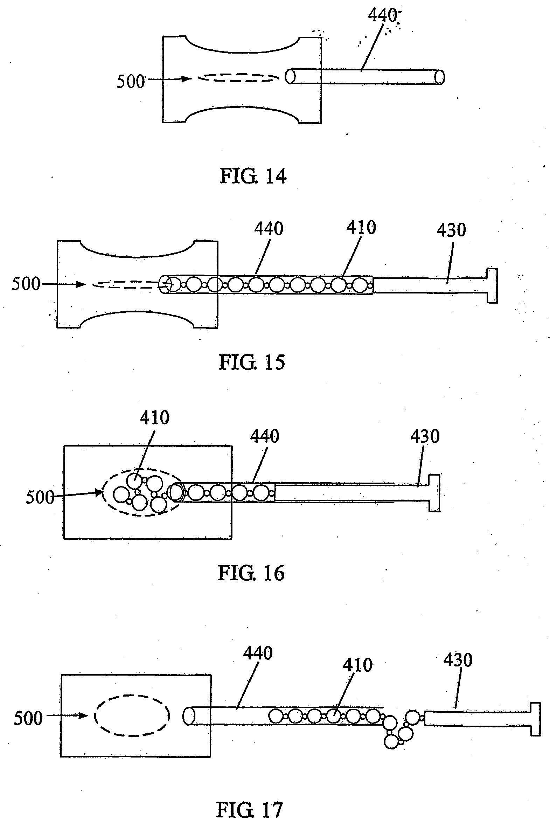

The level (degree) of expansion of the bone cavity is controlled by the volume of the bead filler ("necklace") inserted into the bone cavity. The "necklace" can easily deflect in the bone cavity while impinging against the bone cavity wall. With increasing the volume (or number) of the beads inserted, the bone cavity is expanded and "filled" with the beads. As shown in Figs. 14 to 17, the insertion of the bead filler into a bone cavity 500 can be conducted through a surgical tube 440 and assisted by applying a pressure onto the beads 410 by pushing the stud 430 into the tube 400. When a desired cavity volume is reached, the stud 430 along with its attached "necklace" is withdrawn from the tube 440.

Preliminary results from the clinical trial using a prototype "necklace" device in expanding a compression-fractured vertebral body bone structure prove that this new concept/device works satisfactorily.

**Claims**

1. An orthopaedic paste delivering tool comprising:

> a chamber for storing an orthopaedic paste (80);
> a tube (20) in fluid communication with the chamber storing the paste (80);

a recovery member (30) movably received in a hole provided on a wall of the chamber, which can be pushed to move from a first position to a second position, so that a portion of the recovery member (30) invades the paste (80) stored in the chamber, and thus the paste enters the tube (20) in an amount substantially equivalent to a volume of the invading portion of the recovery member (30); and

the recovery member (30) does not enter said tube (20) or is only partly inserted into said tube (20) **characterized by**

a press member (50) in contact with the paste (80) stored in the chamber, which presses the paste stored in the chamber to eliminate a space created in the chamber when the recovery member (30) is being pulled from the second position to the first position.

2. The tool as defined in claim 1, wherein the recovery member (30) is a rod having a diameter less than 20 times of an inner diameter of the tube (20).

3. The tool as defined in claim 2, wherein the rod has a diameter ranging from 5 times of the inner diameter of the tube (20) to smaller than the inner diameter of the tube (20).

4. The tool as defined in claim 1, wherein the press member (50) is a plate slidably received in the chamber, and one side of the plate is in contact with the paste (80) stored in the chamber and the other side of the plate is adapted to receive a positive pressure source.

5. The tool as defined in claim 4, wherein weight of the plate and/or the positive pressure source contribute a drive to said pressing.

6. The tool as defined in claim 5, wherein the weight of the plate, the positive pressure source or a combination of them are not enough to cause a significant amount of the paste (80) stored in the chamber entering the tube (20).

7. The tool as defined in claim 1 further comprising a driving means for reciprocally performing the pushing and pulling of the recovery member (30).

8. The tool as defined in claim 7, wherein said driving means comprises a pneumatic cylinder (10).

9. A method for delivering a paste (80) comprising the following steps:

filling a chamber with a paste (80), with which a tube (20) is in fluid communication;

pressing the paste in the chamber with a pressure which is not enough to cause a significant amount of the paste (80) in the chamber entering the tube (20);

reciprocally pushing a recovery member (30) to invade the paste (80) in the chamber and pulling the invading recovery member in the chamber, so that the paste (80) enters the tube (20) in an amount substantially equivalent to a volume of an invading portion of the recovery member as a result of said pushing and the recovery member (30) does not enter said tube (20) or is only partly inserted into said tube (20), and that a space created in the chamber by said pulling is eliminated as a result of said pressing, and thus the paste (80) in the chamber is continually delivered through said tube (20).

10. The method as defined in claim 9, wherein the recovery member (30) is a rod having a diameter less than 20 times of an inner diameter of the tube (20).

11. The method as defined in claim 10, wherein the rod has a diameter ranging from 5 times of the inner diameter of the tube (20) to smaller than the inner diameter of the tube (20).

12. The method as defined in claim 9, wherein said pressing is carried out by using a press member (50) slidably received in the chamber, wherein one side of the press member is in contact with the paste (80) stored in the chamber and the other side of the press member is adapted to receive a positive pressure source:

13. The method as defined in claim 12, wherein said press member (50) is a plate.

14. The method as defined in claim 13, wherein weight of the plate and/or the positive pressure source contribute a drive to said pressing.

15. The method as defined in claim 9, wherein said reciprocally pushing and pulling driving is carried out by using a pneumatic cylinder (10).

16. The method as defined in claim 9, wherein said recovery member (30) is pushed to invade the paste (80) in the chamber without entering the tube (20) during said pushing.

17. The method as defined in claim 9, wherein said paste (80) is a liquid-powder mixture having a liquid to solid ratio by volume from about 0.1 to 10.

18. The method as defined in claim 9, wherein said paste (80) is a viscous liquid comprising a polymeric material, which has a viscosity greater than about 500 centipoise.

**Patentansprüche**

1. Abgabeinstrument für orthopädische Paste, das umfasst:

   eine Kammer zum Speichern einer orthopädischen Paste (80);
   ein Rohr (20) in Fluidkommunikation mit der Kammer, die die Paste (80) speichert;
   ein Wiederauffüllungselement (30), das in einem Loch bewegbar aufgenommen wird, das an einer Wand der Kammer bereitgestellt wird, das geschoben werden kann, um sich von einer ersten Position in eine zweite Position zu bewegen, so dass ein Teil des Wiederauffüllungselements (30) in die in der Kammer gespeicherten Paste (80) eindringt, wobei damit die Paste in einem Umfang in das Rohr (20) gelangt, der im Wesentlichen zu einem Volumen des eindringenden Teils des Wiederauffüllungselements (30) äquivalent ist; und
   das Wiederauffüllungselement (30) nicht in das Rohr (20) gelangt oder nur teilweise in das Rohr (20) eingeführt wird,
   **gekennzeichnet durch**
   ein Druckelement (50) in Kontakt mit der in der Kammer gespeicherten Paste (80), das die in der Kammer gespeicherte Paste drückt, um einen Leerraum zu beseitigen, der in der Kammer erzeugt wird, wenn das Wiederauffüllungselement (30) von der zweiten Position in die erste Position gezogen wird.

2. Instrument nach Anspruch 1, wobei das Wiederauffüllungselement (30) ein Stab mit einem Durchmesser von weniger als dem 20-fachen des Innendurchmessers des Rohrs (20) ist.

3. Instrument nach Anspruch 2, wobei der Stab einen Durchmesser hat, der vom 5-fachen des Innendurchmessers des Rohrs (20) bis kleiner als der Innendurchmesser des Rohrs (20) reicht.

4. Instrument nach Anspruch 1, wobei das Druckelement (50) eine Platte ist, die gleitend in der Kammer aufgenommen wird, wobei eine Seite der Platte mit der in der Kammer gespeicherten Paste (80) in Kontakt ist und die andere Seite der Platte angepasst ist, um ein Überdruckquelle aufzunehmen.

5. Instrument nach Anspruch 4, wobei das Gewicht der Platte und/oder der Überdruckquelle beim Antrieb auf das Drücken mitwirkt.

6. Instrument nach Anspruch 5, wobei das Gewicht der Platte, der Überdruckquelle oder einer Kombination davon nicht ausreichen, um zu bewirken, dass eine signifikante Menge der in der Kammer gespeicherten Paste (80) in das Rohr (20) gelangt.

7. Instrument nach Anspruch 1, das des Weiteren eine Antriebseinrichtung zum wechselweisen Durchführen von Drücken und Ziehen des Wiederauffüllungselements (30) umfasst.

8. Instrument nach Anspruch 7, wobei die Antriebseinrichtung einen pneumatischen Zylinder (10) umfasst.

9. Verfahren zur Abgabe einer Paste (80), das die folgenden Schritte umfasst:

   Füllen einer Kammer mit einer Paste (80), mit der sich ein Rohr (20) in Fluidkommunikation befindet;
   Drücken der Paste in der Kammer mit einem Druck, der nicht ausreicht, um zu bewirken, dass eine signifikante Menge der Paste (80) in die Kammer in das Rohr (20) gelangt;

Wechselweises Drücken eines Wiederauffüllungselements (30), um in die Paste (80) in der Kammer einzudringen, und Ziehen des eindringenden Wiederauffüllungselements in der Kammer, so dass die Paste (80) in das Rohr (20) in einem Umfang, der zu einem Volumen eines eindringenden Teils des Wiederauffüllungselements im Wesentlichen äquivalent ist, infolge des Drückens gelangt und das Wiederauffüllungselement (30) nicht in das Rohr (20) gelangt oder nur teilweise in das Rohr (20) eingeführt wird, und dass ein in der Kammer erzeugter Leerraum durch das Ziehen infolge des Drückens beseitigt wird und damit die Paste (80) in der Kammer kontinuierlich durch das Rohr (20) abgegeben wird.

**10.** Verfahren nach Anspruch 9, wobei das Wiederauffüllungselement (30) ein Stab mit einem Durchmesser von weniger als dem 20-fachen des Innendurchmessers des Rohrs (20) ist.

**11.** Verfahren nach Anspruch 10, wobei der Stab einen Durchmesser hat, der vom 5-fachen des Innendurchmessers des Rohrs (20) bis kleiner als der Innendurchmesser des Rohrs (20) reicht.

**12.** Verfahren nach Anspruch 9, wobei das Drücken durch Verwendung eines Druckelements (50) ausgeführt wird, das in der Kammer gleitend aufgenommen wird, wobei eine Seite des Druckelements mit der in der Kammer gespeicherten Paste (80) in Kontakt ist und die andere Seite des Druckelements angepasst ist, um ein Überdruckquelle aufzunehmen.

**13.** Verfahren nach Anspruch 12, wobei das Druckelement (50) eine Platte ist.

**14.** Verfahren nach Anspruch 13, wobei das Gewicht der Platte und/oder der Überdruckquelle beim Antrieb auf das Drücken mitwirkt.

**15.** Verfahren nach Anspruch 9, wobei der wechselweise Druck- und Ziehantrieb mittels eines pneumatischen Zylinders (10) ausgeführt wird.

**16.** Verfahren nach Anspruch 9, wobei das Wiederauffüllungselement (30) geschoben wird, um in die Paste (80) in der Kammer einzudringen, ohne während des Drückens in das Rohr (20) zu gelangen.

**17.** Verfahren nach Anspruch 9, wobei die Paste (80) ein Flüssigkeits-Pulvergemisch mit einem Volumenverhältnis von Flüssigkeit zu Feststoff von etwa 0,1 zu 10 ist.

**18.** Verfahren nach Anspruch 9, wobei die Paste (80) eine viskose Flüssigkeit mit einem Polymermaterial ist, die eine Viskosität größer als etwa 500 Zentipoise hat.

**Revendications**

**1.** Outil de distribution de pâte orthopédique comprenant :

une chambre de stockage d'une pâte orthopédique (80) ;
un tube (20) en communication fluide avec la chambre de stockage de la pâte (80) ;
un élément de récupération (30) réceptionné de manière mobile dans un trou prévu sur une paroi de la chambre, qui peut être poussé pour se déplacer depuis une première position vers une seconde position, de sorte qu'une partie de l'élément de récupération (30) envahisse la pâte (80) stockée dans la chambre, et ainsi que la pâte pénètre dans le tube (20) en une quantité sensiblement équivalente au volume de la partie d'invasion de l'élément de récupération (30) ; et
l'élément de récupération (30) ne pénètre pas dans ledit tube (20) ou est uniquement inséré partiellement dans ledit tube (20) **caractérisé par**
un élément de compression (50) en contact avec la pâte (80) stockée dans la chambre, qui comprime la pâte stockée dans la chambre pour éliminer un espace créé dans la chambre lorsque l'élément de récupération (30) est tiré depuis la seconde position vers la première position.

**2.** Outil tel que défini selon la revendication 1, dans lequel l'élément de récupération (30) est une tige ayant un diamètre inférieur à 20 fois un diamètre interne du tube (20).

**3.** Outil tel que défini selon la revendication 2, dans lequel la tige présente un diamètre s'étendant de 5 fois le diamètre interne du tube (20) jusqu'à plus petit que le diamètre interne du tube (20).

4. Outil tel que défini selon la revendication 1, dans lequel l'élément de compression (50) est une plaque réceptionnée de manière à pouvoir coulisser dans la chambre, et un côté de la plaque se trouve en contact avec la pâte (80) stockée dans la chambre et l'autre côté de la plaque est adapté pour recevoir une source de pression positive.

5. Outil tel que défini selon la revendication 4, dans lequel le poids de la plaque et/ou la source de pression positive contribue à un entraînement de ladite compression.

6. Outil tel que défini selon la revendication 5, dans lequel le poids de la plaque, la source de pression positive ou une combinaison de ceux-ci ne sont pas suffisants pour provoquer l'entrée d'une quantité significative de la pâte (80) stockée dans la chambre dans le tube (20).

7. Outil tel que défini selon la revendication 1 comprenant en outre un moyen d'entraînement pour exécuter de manière réciproque la poussée et la traction de l'élément de récupération (30).

8. Outil tel que défini selon la revendication 7, dans lequel ledit moyen d'entraînement comprend un cylindre pneumatique (10).

9. Procédé de distribution d'une pâte (80) comprenant les étapes suivantes :

remplissage d'une chambre avec une pâte (80), avec laquelle un tube (20) se trouve en communication fluide ; compression de la pâte dans la chambre avec une pression qui n'est pas suffisante pour conduire une quantité significative de pâte (80) dans la chambre à entrer dans le tube (20) ; pousser de manière réciproque un élément de récupération (30) pour remplir la pâte (80) dans la chambre et tirer l'élément de récupération d'invasion dans la chambre, de sorte que la pâte (80) pénètre dans le tube (20) en une quantité sensiblement équivalente à un volume d'une partie d'invasion de l'élément de récupération en résultat de ladite poussée et l'élément de récupération (30) ne pénètre pas ledit tube (20) ou est uniquement partiellement inséré dans ledit tube (20), et en ce qu'un espace créé dans la chambre par ladite traction est éliminé en résultat de ladite compression, et ainsi la pâte (80) dans la chambre est continuellement distribuée à travers ledit tube (20).

10. Procédé tel que défini selon la revendication 9, dans lequel l'élément de récupération (30) est une tige ayant un diamètre inférieur à 20 fois un diamètre interne du tube (20).

11. Procédé tel que défini selon la revendication 10, dans lequel la tige présente un diamètre s'étendant de 5 fois le diamètre interne du tube (20) jusqu'à plus petit que le diamètre interne du tube (20).

12. Procédé tel que défini selon la revendication 9, dans lequel ladite compression est conduite en utilisant un élément de compression (50) reçu de manière à pouvoir coulisser dans la chambre, où un côté de l'élément de compression se trouve en contact avec la pâte (80) stockée dans la chambre et l'autre côté de l'élément de compression est adapté pour recevoir une source de pression positive.

13. Procédé tel que défini selon la revendication 12, dans lequel ledit élément de compression (50) est une plaque.

14. Procédé tel que défini selon la revendication 13, dans lequel le poids de la plaque et/ou la source de pression positive contribuent à un entraînement de ladite compression.

15. Procédé tel que défini selon la revendication 9, dans lequel ledit entraînement réciproque de poussée et de traction est conduit en utilisant un cylindre pneumatique (10).

16. Procédé tel que défini selon la revendication 9, dans lequel ledit élément de récupération (30) est poussé pour remplir la chambre de la pâte (80) sans pénétrer le tube (20) durant ladite poussée.

17. Procédé tel que défini selon la revendication 9, dans lequel ladite pâte (80) est un mélange de liquide-poudre ayant un rapport liquide à solide en volume d'environ 0,1 à 10.

18. Procédé tel que défini selon la revendication 9, dans lequel ladite pâte (80) est un liquide visqueux comprenant un matériau polymère, qui présente une viscosité supérieure à environ 500 centipoises.

FIG. 2
(PRIOR ART)

FIG. 1
(PRIOR ART)

FIG. 3

$F_2$  Filling
Force

50

40

20

30

$F_1$
Injection
Force

81

80

FIG. 4

10

Frequency
and
Number of
Stokes
Command

Power
Supply

60

13

Air Supply

FIG. 6

FIG 5

FIG. 7

200

210

FIG. 8

200

210

220

FIG. 9

310

320

FIG. 10

310

300

FIG. 11

420

410

430

FIG. 12

410b

410a

430

FIG. 13

FIG. 14

FIG. 15

FIG. 16

FIG. 17

**REFERENCES CITED IN THE DESCRIPTION**

**Patent documents cited in the description**

- WO 9831292 A
- US 20050113843 A1
- US 20040174768 A1
- US 6033105 A
- WO 0113822 A1
- US 20040186481 A1